# EUROPÄISCHE PATENTSCHRIFT

(11) **EP 2 050 422 B1**
(45) Veröffentlichungstag und Bekanntmachung des Hinweises auf die Patenterteilung: **30.06.2010**
(21) Anmeldenummer: 08075823.8
(22) Anmeldetag: 14.10.2008
(51) Int. Cl.: A61F 13/04, A61F 5/01

(54) **Orthopädische Formstütze mit einer fraktalen Stützstruktur nach biologischem Vorbild**
Orthopaedic shaped support with a fractal support structure using biology as a pattern
Appui moulé orthopédique doté d'une structure d'appui fractale selon un modèle biologique

(30) Priorität: 16.10.2007 DE 202007014631 U
(43) Veröffentlichungstag der Anmeldung: 22.04.2009
(73) Patentinhaber: Stiftung Alfred-Wegener-Institut für Polar- und Meeresforschung, 27570 Bremerhaven (DE)
(72) Erfinder: Hamm-Dubischar, Christian, 27580 Bremerhaven (DE); Cuma, Kara, 27570 Bremerhaven (DE)

(56) Entgegenhaltungen:
- DE-T2- 69 828 024
- US-A1- 2003 171 703
- US-B1- 6 673 029

## Beschreibung

Die Erfindung bezieht sich auf eine orthopädische Formstütze zur nicht-chirurgischen Behandlung von Knochen, Gelenken, Sehnen oder Bändern, hergestellt aus einer aus einem thermisch umformbaren Material bestehenden, durchbrochenen Leichtbauplatte mit einer mechanisch verstärkenden, regelmäßig periodisch aufgebauten Stützstruktur nach biologischem Vorbild, die an das zu behandelnde Körperteil angepasst und mittels einer Fixiereinrichtung fixiert wird.

Der Bereich Therapie und Immobilisation auf dem Gebiet der Medizin hat eine Wachstumsrate von jährlich 5 %. Einige Gründe für die Zunahme dieser Zahl liegen in den Sportverletzungen, aber auch in den steigenden altersbedingten Frakturen aufgrund des demographischen Wandels. Technische Innovationen sind gefragt, um auf dem auf dem auf 600 Mio. Euro geschätzten Markt in Europa konkurrenzfähig zu sein. Es ist anzunehmen, dass der Bedarf medizintechnischer Produkte im Bereich der Orthopädie für Orthosen, Bandagen und elastische Binden zunimmt. Bei Frakturen, Bänderrissen, Gelenkentzündungen, die am Menschen (oder auch am Tier) auftreten, sowie andere zahlreiche Krankheiten ist der Einsatz von stabilisierenden Verbänden notwendig. In allen Lebenslagen besteht die Gefahr von Verletzungen durch Unfälle, wie z.B. Frakturen, Prellungen, Verstauchungen und vielen anderen Fällen, die eine Ruhigstellung erfordern. Für diese Art der Ruhigstellung wird in der Medizin auf Stützverbände zurückgegriffen. Konkret werden überwiegend Gips- und Kunststoffverbände eingesetzt. Innovative Produkte, wie der Glasfaser Castverband, werden aufgrund ihres geringen Gewichtes und des damit verbundenen Tragekomfort dabei zunehmend eingesetzt. Ziel der hier angeführten Erfindung ist es, möglichst vielen Patienten durch eine effektive technische Entwicklung zu helfen und das eingeschränkte Wohlbefinden, das zum Beispiel durch Unfälle verursacht worden ist, zu verbessern oder über die Zeit der Verletzung besser ertragbar zu machen. Die Entwicklung neuer Stützverbände mit gutem Tragekomfort kann hier einen Beitrag leisten.

Bei orthopädischen Formstützen handelt es sich um so genannte "Orthesen", um eine industriell oder durch Orthopädietechniker hergestellte technische Hilfe, die zur Unterstützung von eingeschränkt funktionstüchtigen Körperteilen zum Einsatz gebracht wird. Es gibt Orthesen zur Fixation von nicht reversiblen oder reversiblen Fehlstellungen. Weiterhin gibt es Orthesen, die zur zeitweiligen Ruhestellung von Körperteilen eingesetzt werden. Andere haben die Aufgabe, Entlastung vorzunehmen, wie beispielsweise Rumpforthesen (aktiv oder passiv) oder orthopädische Korsetts. Grundsätzlich sichern Orthesen Körperfunktionen und sorgen für die gewünschte Stabilität. Im Gegensatz dazu werden Bandagen eingesetzt, wenn die absolute mechanische Sicherheit nicht unbedingt notwendig ist. Neben vollfunktionellen Therapien werden noch semifunktionelle Therapien - in der Regel als Alternative zu einer operativen Behandlung - durchgeführt, bei der die verwendete Orthese aus einem semirigiden, thermoplastisch verformbaren Material gefertigt ist und einen geringeren Bewegungsspielraum zulässt als zum Beispiel U-förmige Orthesen.

### STAND DER TECHNIK

Grundsätzlich ist es aus der US 4.683.877 bekannt, ein faserverstärktes Plattenmaterial aus einem thermoplastischen Kunstharz an die zu behandelnde Körperstelle anzupassen und mit Fixiersystemen, beispielsweise röntgenstrahlendurchlässige Clips in Kombination mit Stretchbandagen, zu halten. Eine faserverstärkte Leichtbauplatte mit eine maximalen Flächengewicht von 1000 g/m² und einer vorgegebenen Biegesteifigkeit für eine orthopädische Formstütze mit einem triaxialen (wabenförmigen) Faserverlauf ist aus der DE 698 28 024 T2 bekannt. Geformte Halbschalen werden durch eine Umwicklung mit einer elastischen Bandage fixiert. Dabei weist die Leichtbauplatte Durchbrüche auf, die bis zu 33% der Gesamtfläche einnehmen können. Eine mit einem weichen und flexiblen Harz bedeckte Kante verhindert ein Einschnüren in den Körperteil beim Tragen. Aus der US 2004/0024337 A1 ist eine Leichtbauplatte für eine orthopädische Formstütze bekannt, die aus einem Substrat aus einem thermoplastischen Kunstharz und einer Polsterung besteht. Die Substratplatte weist kleine Durchbrüche für eine Durchlüftung auf. Die Polsterung besteht aus schmalen Streifen, die zwischen den Durchbrüchen verlaufen. Die Leichtbauplatte kann kantenüberlappend an das zu fixierende Körperteil angepasst werden. Eine ähnlich gelöcherte Leichtbauplatte für eine orthopädische Formstütze ist aus der US 6.093,161 bekannt. Hier kann der Kantenverschluss mittels Reißverschluss oder Klettbänder erfolgen. Eine orthopädische Formstütze in Leichtbauweise mit einem Skelettaufbau aus dreieckigen Durchbrüchen und Stegen ist aus der US 5.836.902 bekannt. Diese Formstütze ist vorgeformt, weist eine Scharnierseite oder Überlappungsseite (die mit in Löcher eingreifenden Stiften fixiert wird) auf und kann auf der anderen Seite mit Klettband oder Clipsen geschlossen werden.

Der Stand der Technik, von dem die vorliegende Erfindung als nächstliegend ausgeht, wird in der US 6.942.628 B1 offenbart. Gezeigt wird eine orthopädische Formstütze zur nicht-chirurgischen Behandlung von Knochen, Gelenken, Sehnen oder Bändern, die aus einer aus einem thermisch umformbaren Material bestehenden, durchbrochenen Leichtbauplatte hergestellt ist, die an das zu behandelnde Körperteil angepasst und mittels einer Fixiereinrichtung fixiert wird. Dabei weist die Leichtbauplatte eine mechanisch verstärkende, regelmäßig periodisch aufgebaute Stützstruktur nach biologischem Vorbild auf. Speziell handelt es sich um eine Wabenstruktur als optimierte Strukturgeometrie, dabei besteht die Wabenstruktur aus geschlossenen Rippen ohne Wabenboden, der von sechseckigen Durchbrüchen gebildet wird. Eine Aussage über die Größe der Durchbrüche wird nicht getroffen. Die Wabenstruktur kann entweder aus einem Mehrschichtaufbau oder aus mit Mikroperlen gefüllten, im Querschnitt sechseckigen Wabenrippen aus einem glasfaserverstärkten, aushärtbaren Kunstharz bestehen. Die zweite Alternative ist in der US 6.673.029 B1 geschützt. Da aber beide Ausführungsformen eine konstante Gewichtsverteilung über den Querschnitt aufweisen, handelt es sich nicht um eine Leichtbauplatte mit einem hohen Flächenträgheitsmoment, sondern um einfach einfache Leichtbauplatte mit einfacher Wabenstruktur.

Nach einer Anpassung an das zu behandelnde Körperteil wird die entsprechend zurechtgeschnittene Leichtbauplatte aus einem aushärtbaren Kunststoff durch Wärmeeinwirkung verfestigt. Verschlossen werden die beiden Längskanten der Leichtbauplatte nach der Umformung beispielsweise durch Clips oder durch einen Sägezahnverschluss bei überlappenden Kanten. Die bekannte Leichtbauplatte weist zwar bereits eine an die Natur angepasst Geometrie in einer einzigen Ebene auf, diese ist jedoch relativ einfacher Ausgestaltung, sodass nicht für jeden Anwendungsfall eine optimierte Kraftverteilung und damit Leichtbaukonstruktion der orthopädischen Leichtbaustütze gewährleistet werden kann. Insbesondere liegt die gesamte Stützstruktur direkt auf der zu behandelnden Körperstelle auf.

### AUFGABENSTELLUNG

Ausgehend von der oben gewürdigten, der Erfindung nächstliegenden Druckschrift ist die **AUFGABE** für die vorliegende Erfindung darin zu sehen, die eingangs beschriebene gattungsgemäße orthopädische Formstütze in ihrer Stützstruktur nach biologischem Vorbild so weiterzubilden, dass eine optimale Anpassung an den jeweiligen Anwendungsfall möglich ist. Dabei soll eine weitere Gewichtsreduzierung durch Materialeinsparung bei gleichzeitig guter Modellierbarkeit der Leichtbauplatte erreicht werden. Gleichzeitig sollen trotz hoher Stabilität die Berührungsflächen mit der zu behandelnden Stelle minimiert werden. Die Vorteile der bekannten orthopädischen Formstütze, wie Atmungsaktivität und Sicht auf die Hautoberfläche sollen erhalten bleiben bzw. weiter verbessert werden. Die **LÖSUNG** für diese Aufgabe ist dem Hauptanspruch zu entnehmen. Vorteilhafte Weiterbildungen der Erfindung werden in den Unteransprüchen aufgezeigt und im Folgenden im Zusammenhang mit der Erfindung näher erläutert.

Bei der erfindungsgemäßen orthopädischen Formstütze (charakterisierender Name "CellFix") ist eine fraktale Gliederung der Stützstruktur in zumindest zwei fraktalen Ebenen vorgesehen, die auf der der zu behandelnden Körperstelle zugewandten Seite der Leichtbauplatte bündig abschließen. Bei einer fraktalen Gliederung der Stützstruktur in mehreren, zumindest zwei fraktalen Ebenen wiederholt sich der Verlauf der Stützkontur in sich selbst zumindest noch ein weiteres Mal. Die Anzahl der jeweils in der Regel um eine Dimension kleiner werdenden, selbstähnlichen Wiederholungen gibt die Anzahl der fraktalen Ebenen an. Zwischen den Ebenen sind die Übergänge fließend und integriert. Wichtig für die vorliegende Erfindung ist dabei, dass trotz einer sehr hohen Festigkeit bei minimalem Materialeinsatz auch die Berührungsfläche mit der zu behandelnden Körperstelle minimiert wird., Durch die fraktale Strukturierung entsteht eine Materialverteilung senkrecht zur Wabenplatte (z-Richtung), die einen hohen Anteil an Material weit entfernt von der neutralen Faser aufweist (entsprechend einem T-Träger). Das dadurch entstehende hohe Flächenträgheitsmoment trägt wesentlich zur Steifigkeit der Struktur bei und erlaubt auch den effektiven Einsatz von Materialien mit geringen E-Moduli, wie z.B. Polyvinylalkohol (PVA, siehe unten). Durch den bündigen Abschluss der zumindest beiden Ebenen auf der der zu behandelnden Körperstelle zugewandten Seite der Leichtbauplatte, also in der Regel auf der Innenseite der orthopädischen Formstütze, ist sicher gewährleistet, dass die Kraft über die gesamte Stützstruktur in die zu behandelnde Körperstelle eingeleitet wird, sodass keine Irritationen durch Überbelastungen entstehen.

Als weitere wesentliche Vorteile der Verwendung einer fraktalen Stützstruktur sind anzusehen, dass Kerbspannungsspitzen durch die direkten, fließenden Übergänge (in z-Richtung) zwischen den integrierten Strukturen in den verschiedenen fraktalen Ebenen der Stützstruktur ohne Kerb- oder Bruchkanten sicher verhindert werden und dass bei gleicher Festigkeit leichtere, effizienter stützende Strukturen eingesetzt werden können. Die Festigkeit ergibt sich durch entsprechende Ausprägung der fraktalen Stützstruktur sowohl in kleinen Stützbereichen als auch in größeren Stützbereichen und in ganz großen Stützbereichen. Die Gliederung der Stützstruktur wächst also mit der Größe des Stützbereichs und lässt sich einfach daran anpassen. Außerdem kann die Struktur aus einem Guss gefertigt werden, spezielle Verbindungen oder Klebungen und die sich daraus ergebende Probleme entfallen. Eine Leichtbaukonstruktion mit einer seine Oberflächenhaut mechanisch verstärkenden, regelmäßig periodisch aufgebauten Stützstruktur mit einer fraktalen Gliederung der Stützstruktur in zumindest zwei fraktalen Ebenen mit integrierten, fließenden Übergängen zwischen den fraktalen Ebenen wird in der deutschen Patentanmeldung DE 10 2007 011 107.1 beansprucht, deren Priorität bei der vorliegenden Erfindung beansprucht wird. Der Inhalt der älteren deutschen Patentanmeldung soll damit ausdrücklich in den Offenbarungsumfang der vorliegenden Erfindung einbezogen werden.

Der Begriff "Fraktal" (Adjektiv oder Substantiv) wurde von Benoît Mandelbrot (1975) geprägt und leitet sich aus dem Lateinischen von fractus - gebrochen ab. Mit Fraktalen werden natürliche oder künstliche Gebilde oder geometrische Muster bezeichnet, die einen hohen Grad von Skaleninvarianz bzw. Selbstähnlichkeit aufweisen. Das ist beispielsweise der Fall, wenn ein Objekt aus mehreren verkleinerten Kopien seiner selbst besteht. Jede Verkleinerung definiert dann eine fraktale Ebene. Anzutreffen ist immer der fließende Übergang zwischen den Strukturelementen in den verschiedenen fraktalen Ebenen. Geometrische Objekte dieser Art unterscheiden sich in wesentlichen Aspekten von gewöhnlichen glatten Figuren. Dabei muss die Selbstähnlichkeit jedoch nicht perfekt sein, wie die erfolgreiche Anwendung der Methoden der fraktalen Geometrie auf natürliche Gebilde wie Bäume, Wolken, Küstenlinien, etc. zeigen. Die genannten Objekte sind in mehr oder weniger starkem Maß selbstähnlich strukturiert (ein Baumzweig sieht ungefähr so aus wie ein verkleinerter Baum), die Ähnlichkeit ist jedoch nicht streng, sondern stochastisch. Im Gegensatz zu Formen der euklidischen Geometrie, die bei einer Vergrößerung oft flacher und flacher und damit einfacher werden (z.B. Kreis), können bei Fraktalen immer komplexere und neue Details auftauchen.

Fraktale Muster werden oft durch rekursive Operationen erzeugt. Auch einfache Erzeugungsregeln ergeben nach wenigen Rekursionsschritten schon komplexe Muster. Dies ist zum Beispiel am Pythagoras-Baum zu sehen. Ein solcher Baum ist ein Fraktal, welches aus Quadraten aufgebaut ist, die so angeordnet sind wie im Satz des Pythagoras definiert. Ein weiteres Fraktal ist das Newton-Fraktal, es wird berechnet über das Newton-Verfahren, das zur Nullstellenberechnung verwendet wird. Ein Fraktal im dreidimensionalen Raum ist der Menger-Schwamm. Fraktale Konzepte finden sich auch in der Natur. Dabei ist jedoch die Anzahl der Ebenen von selbstähnlichen Strukturen begrenzt und beträgt oft nur 3-5. Typische Beispiele aus der Biologie sind die streng fraktalen Strukturen bei der grünen Blumenkohlzüchtung Romanesco, bei den Farnen und bei den Ammoniten. Aber auch Mikroalgen zeigen oft fraktal aufgebaute Stützstrukturen in mehreren Ebenen. Weit verbreitet sind auch fraktale Strukturen mit statistischer Selbstähnlichkeit. Dazu zählen beispielsweise Bäume, der Blutkreislauf, Flusssysteme und Küstenlinien.

Die erfindungsgemäße Formstütze mit ihrer speziellen fraktalen Gliederung der Stützstruktur erfüllt einerseits ihre medizinische Funktion und wirkt andererseits noch in einem Höchstmaße ästhetisch. Sie zeigt eine permeable Leichtbauweise mit guten Festigkeitseigenschaften und weist noch weitere positive Eigenschaften wie gutes Handling und relativ geringe Herstellungskosten auf. Gleichzeitig wird mit dem hochspeziellen Design der Formstütze den Ärzten, Krankenschwestern und Pflegepersonal eine Vereinfachung geschaffen, sodass diese ihre Arbeit besser durchführen können. Die Arbeit wird insbesondere auch dadurch erleichtert, dass durch eine bevorzugte Größenwahl der Durchbrüche in der Form, dass alle Durchbrüche eine Größe unterhalb von 30 mm aufweisen, mit hoher Sicherheit Ödembildungen vermieden werden. Dabei bringt es die fraktale Gliederung mit sich, dass große Durchbrüche von einer Vielzahl von ähnlich geformten Durchbrüchen noch unterteilt werden. Zur Vermeidung von Ödemen ist es daher vorteilhaft, wenn alle Durchbrüche einen Durchmesser von unter 30 mm aufweisen, dabei können die größten Durchbrüche einen Durchmesser knapp unterhalb von 30 mm aufweisen. Dennoch kann durch das sehr gute Verhältnis von Gewicht zu Steifigkeit eine hohe Materialeinsparung gegenüber bekannten orthopädischen Formstützen bei gleichzeitig hoher Stabilität, bzw. eine erhöhte Stabilität bei vergleichbarem Materialeinsatz erzielt werden.

Bevorzugt wird bei der Erfindung eine fraktale Gliederung der Stützstruktur nach dem Vorbild der biomineralisierenden Exoskelette mariner Mikroalgen oder Ammoniten gewählt. Um eine hervorragende Leichtbauweise bei der erfindungsgemäßen Formstütze zu erzielen, werden bevorzugt marine Exoskelette der Kieselalgen zur Betrachtung herangezogen. Diese kleinen Organismen weisen bei hoher Durchlässigkeit optimierte Schalenstrukturen auf, die durch unterschiedliche Form- und Gestaltgebung eine Leichtbauweise ermöglichen. Hilfreich bei der Lösung des Gewichts- und Permeabilitätsproblems und bei der anschließenden Skalierung der natürlichen Stützstrukturen auf die Abmaße der Stützstruktur der erfindungsgemäßen Formstütze ist ein Verfahren zur Erzeugung von konstruktiven Erstmodelldaten gemäß der DE 103 56 682 A1 (der Inhalt dieser Patentanmeldung soll ebenfalls durch das Zitat zum Offenbarungsgehalt der beanspruchten Erfindung gehören) mit den wesentlichen Verfahrensschritten:
1. Bereitstellung der relevanten Grundparameter der zu erstellenden technischen Leichtbaustruktur in Bezug auf die technische Problemstellung,
2. Vorauswahl von einem oder mehreren biomineralisierten Einzellern mit zu den bereitgestellten Grundparametern passenden natürlichen Schalenarchitekturen,
3. Auswahl von einer oder mehreren Feinstrukturen der für eine technische Realisierung aussichtsreichsten Bereiche der vorausgewählten Schalenarchitekturen,
4. direkte Abnahme der konstruktiven Daten von den ausgewählten Feinstrukturen,
5. Skalierung der abgenommenen konstruktiven Daten auf die Grundparameter der zu erstellenden technischen Leichtbaustruktur,
6. Kombination und Anpassung der skalierten konstruktiven Daten zu eine Satz von Erstmodelldaten für ein Erstmodell der zu erstellenden technischen Leichtbaustruktur und
7. Optimierung des Erstmodells.

Die Ausbildung der fraktalen Gliederung der Stützstruktur ist in zahlreichen Varianten möglich. Als bevorzugt soll genannt werden einen fraktale Gliederung aus Stützrippen mit geradem oder gewelltem Verlauf. Die Vorteile von Stützrippen in der Leichtbautechnik sind hinlänglich bekannt. Weiterhin kann eine Kombination der fraktal gegliederten Stützstruktur mit einer nicht fraktal gegliederten Stützstruktur vorgesehen sein, um jeweils speziell belastete Bereich der Leichtbauplatte im späteren Einsatzfall auch entsprechend mehr oder weniger stützend strukturieren zu können.

Bevorzugt besteht die Leichtbauplatte aus einem Kunststoff (synthetisches Polymer), der thermoplastische und duroplastische Eigenschaften aufweist. Damit kann die Leichtbauplatte durch Wärmeeinwirkung weich gemacht und umgeformt, d.h. an das zu stützende Körperteil angeformt werden und dann aushärten. Die Erwärmung kann beispielsweise in einfacher Weise in der Mikrowelle erfolgen. Die Aushärtung kann einfach an der Luft oder beispielsweise unter Strahlungseinwirkung erfolgen. Geeignete Kunststoffe sind beispielsweise PVA oder PVC. Weiterhin kann der Kunststoff auch faserverstärkt sein, beispielsweise mittels Glas- oder Kohlenstofffasern (GFK, CFK). Eine weitere bevorzugte Alternative ist auch eine Leichtbauplatte, die mittels Hohlkugeln versteift ist. Hierdurch kann wiederum optimal auf die örtlichen Belastungsanforderungen im jeweiligen Einsatzfall eingegangen werden.

Grundsätzlich sind bei der Erfindung die Berührungsflächen der orthopädischen Formstütze mit der Haut der zu behandelnden Körperstelle durch die Ausbildung von mehreren fraktalen Ebenen gleichmäßig verteilt. Die Berührungsflächen sind dabei so auszuführen, dass sie zu keinen Hautirritationen führen. Diese kann bereits durch die Glätte des Materials der Leichtbauplatte an sich erfüllt sein. Weist diese aber an den Durchbruchöffnungen beispielsweise auch nur geringe Grate auf, ist es vorteilhaft, ein hautverträgliches Polstersystem auf der Unterseite (der der zu behandelnden Körperstelle zugewandten Seite) der Stützstruktur vorzusehen. Dabei kann es sich bei der Ausbildung des Polstersystems um ein textiles oder aufblasbares System handeln. Beispielsweise kann eine dicke, weiche, eventuell pneumatisch ausdehnbare Polsterung den Zwischenraum zwischen der Haut und der orthopädischen Formstütze bei Muskelschwund und bei Schwellungen durch Dehnen bzw. Stauchen ausfüllen. Dabei werden in der Regel flexible, hautverträgliche und einfach zu entsorgende Polstermaterialien verwendet, beispielsweise Naturfasern (Pflanzenfasern: Baumwolle u. a.), Fasern tierischen Ursprungs (Wolle u. a.), Chemiefasern (Fasern aus synthetischen Polymeren: Polyester u. a) oder Fasermischungen (Polyester-Watte). Einfachstenfalls ist eine Mullbinde, die vor dem Anlegen der orthopädischen Stütze auf die zu behandelnde Körperstelle aufgebracht wird, ein bereits ausreichendes Polstersystem, das allerdings den Einblick auf die zu behandelnde Körperstelle verdeckt. Vorteilhaft ist deshalb, wenn eine Anpassung des Polstersystems an die durchbrochene Leichtbauplatte in der Form, dass die Durchbrüche offen bleiben, erfolgt. Bei einem derartigen Polstersystem kann es sich beispielsweise um schmale Schaumstoffstreifen handeln, die auf die Stege, d.h. in der Regel auf die Stützkontur der ersten fraktalen Ebene, aufgeklebt werden. Es kann sich aber beispielsweise auch um eine textile Beflockung handeln, die auf die Unterseite der Stützstruktur aufgesprüht wird.

Eine Anpassung der orthopädischen Formstütze nach der Erfindung erfolgt in der Regel um eine Extremität des Patienten. Durch das "Herumwickeln" der Leichtbauplatte um beispielsweise einen Unterarm bildet sich bevorzugt ein Längsspalt, der durch die beiden Längskanten der Leichtbauplatte nach der Anpassung gebildet ist. Es entsteht eine Stoßstelle zwischen den beiden Längskanten der Leichtbauplatte. Diese ist von Vorteil, weil sie der zu behandelnden Stelle die Möglichkeit der Ausdehnung gibt, sodass auftretende Schwellungen nicht zu Schmerzverursachern werden. Bei konventionellen Gipsverbänden werden diese oft nach ihrem Anlegen aus diesem Grunde einmal der Länge nach gespalten (Spaltgips). Damit die orthopädische Formstütze dann nicht ihre Stützfunktion verliert und ggfs. sogar abfällt, ist es vorteilhaft, wenn die gegenüberliegende Längskanten durch die Fixiereinrichtung, die grundsätzlich bei derartigen orthopädischen Formstützen vorgesehen ist, zusammengehalten werden. Es muss also eine Fixierung erfolgen. Bei den bekannten Spaltgipsen erfolgt diese über ein Umwickeln mit einer elastischen Binde, was bei der Erfindung ebenfalls ohne Probleme möglich ist, sodass eine Kombination mit Kompressionsmaterial auf der Oberseite der Stützstruktur vorteilhaft vorgesehen werden kann. Hierdurch wird aber wiederum der Einblick auf die zu behandelnde Stelle verwehrt. Vorteilhaft ist es deshalb, wenn eine Ausbildung der Fixiereinrichtung als den Längsspalt überbrückender Klettverschluss oder als Haken- oder Clipverschluss mit einem elastischen Bindeneinsatz vorgesehen ist. Nähere Einzelheiten hierzu sind dem speziellen Beschreibungsteil zu entnehmen. Neben dem stumpfen Gegenüberliegen der beiden Längskanten der ungeformten Leichtbauplatte ist es auch möglich, die Längskanten überlappen zu lassen, sodass sie zum Übereinanderliegen kommen. Dabei kann es sich um mit Passform übereinander liegende Längskanten handeln, die durch die Fixiereinrichtung zusammengehalten werden. Bevorzugt kann eine Ausbildung der Passform mit glatten oder gezahnten Längskanten vorgesehen sein, wobei die Fixiereinrichtung als Klettverschluss entlang des Längsspaltes ausgebildet ist. Nähere Einzelheiten sind auch hierzu dem speziellen Beschreibungsteil zu entnehmen

Vorteilhaft kann auch die Farbe der orthopädischen Formstütze nach der Erfindung frei gewählt werden, die Farbe "Weiß" ist nicht zwingend. Gleiches gilt auch für das optional vorgesehene Polstersystem. Dadurch kann die orthopädische Formstütze nach der Erfindung unterschiedlichen Altersgruppen interessant und damit leichter tragbar gemacht werden.

Zusammenfassend gesehen bietet die orthopädische Formstütze nach der Erfindung folgende Vorteile:
- hochpermeable stabile Leichtbaukonstruktion als Exoskelett/Stützverband
- "echte" Leichtbaukonstruktion mit einem hohen Flächenträgheitsmoment, d.h. mit einer Masseverteilung ähnlich einem I-Träger
- hoher mechanischer Halt
- medizinische Vorteile durch erhöhte Atmungsaktivität
- erhöhter Tragekomfort durch reduziertes Gewicht sowie erhöhte Atmungsaktivität
- Kombinierbarkeit mit unterschiedlichen Materialien/Farben für unterschiedliche Anwendungen
- Warm-/Heißumformung möglich, schnelle Abkühlung
- Kombinierbarkeit mit Kompressionsmaterial (elastische Binde) möglich, dadurch semifunktionelle Immobilisierung und Verhinderung von Ödemen
- Einsatz für vollständige Immobilisierung (entsprechend Gipsverband) möglich
- Kombination mit an die offene Struktur angepassten Polstersysteme möglich, die vollkommen offen gestaltet sind
- außergewöhnliches, interessantes und vielfach modifizierbares Design
- Nutzung bionischer, fraktaler Schalenversteifungen für ein medizinisches Exoskelett, dabei Nutzung der Möglichkeit, einen prozentual sehr hohen Flächenanteil der Struktur völlig offen zu gestalten
- Kombinierbarkeit mit umformbaren, eingeführten Materialien.
- Kombinierbarkeit mit technischen Hochleistungsmaterialien
- echter Leichtbau durch hohes Flächenträgheitsmoment (in der Summe Querschnitt entsprechend T-Träger)
- Größe aller Durchgänge unter 30 mm möglich, dadurch werden Ödeme verhindert, dennoch ist eine Nutzung der maximalen Porendurchmesser möglich.

### AUSFÜHRUNGSBEISPIELE

Ausbildungsformen der orthopädischen Formstütze nach der Erfindung werden nachfolgend anhand der schematischen Figuren zum weiteren Verständnis der Erfindung näher erläutert. Dabei zeigt:
- **FIGUR 1**: ein Foto einer konkreten Ausführungsform der orthopä- dischen Formstütze auf Basis von marinen Exoskeletten,
- **FIGUR 2**: das 3-D Volumenmodell der Leichtbauplatte für die orthopädische Formstütze gemäß Figur 1,
- **FIGUR 3**: ein Fixierungssystem mit Klettverschluss,
- **FIGUR 4**: ein Fixierungssystem mit Hakenverschluss,
- **FIGUR 5**: ein Fixierungssystem mit Clipverschluss,
- **FIGUR 6**: ein Fixierungssystem mit Klettverschluss bei glatter Passform und
- **FIGUR 7**: ein Fixierungssystem mit Klettverschluss bei gezahnter Passform.

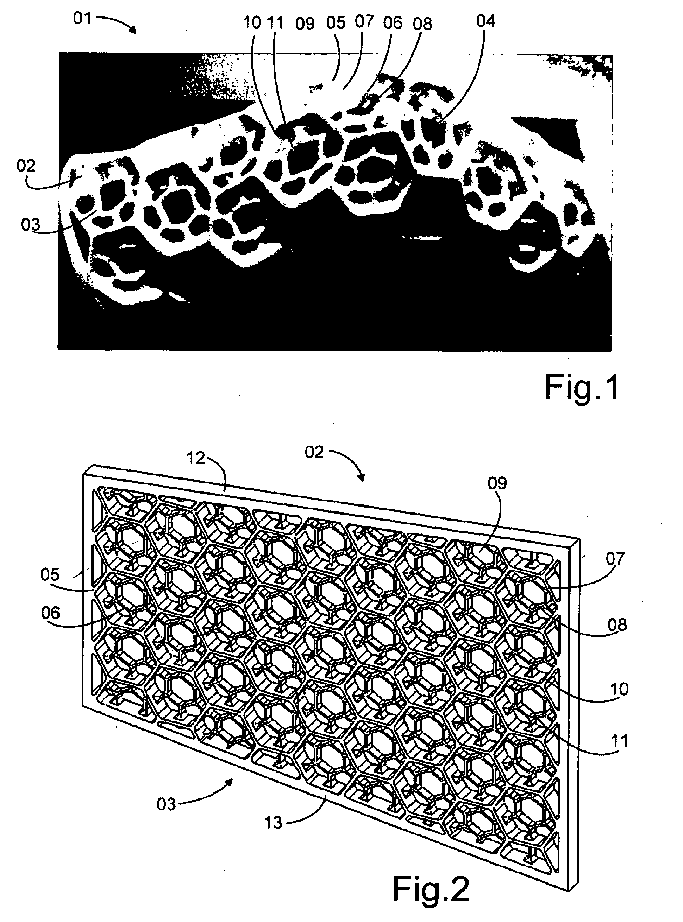

Die **FIGUR 1** zeigt ein Foto einer konkreten orthopädische Formstütze **01** (im Ausführungsbeispiel aus PVA mit einem Gesamtgewicht von nur extrem geringen ca. 62g) nach der Erfindung zur nicht-chirurgischen Behandlung von Knochen, Gelenken, Sehnen oder Bändern. Diese wird hergestellt aus einer Leichtbauplatte **02,** die aus einem thermisch umformbaren Material besteht. Die Leichtbauplatte **02** weist eine mechanisch verstärkende, regelmäßig periodisch aufgebaute fraktale Stützstruktur **03** nach biologischem Vorbild auf, die Durchbrüche **04** aufweist.

In der **FIGUR 1** ist die Passform der orthopädischen Formstütze **01** zu erkennen, die für die Immobilisation eines menschlichen Armes im Bereich des Ellenbogengelenks vorgesehen ist. Diese Passform kann erreicht werden, wenn die Leichtbauplatte **02** erwärmt und im thermoplastischen Zustand an den Arm angeformt wird. Das anschließende Abkühlen bewirkt dann eine dauerhafte Aushärtung der Leichtbauplatte **02** in der angepassten Umformung. Die Stoßstelle der beiden Längskanten der Leichtbauplatte **02** liegen auf der Unterseite der orthopädischen Formstütze **01** und sind in der **FIGUR 1** nicht zu erkennen (sie können einander gegenüberliegen oder überlappen). Die Umformung ist reversibel und kann mehrfach erfolgen. Unter erneuter Erwärmung können andere Anpassungen vorgenommen werden. Die thermoplastischen und duroplastischen Eigenschaften der verwendeten Polymere bleiben dauerhaft erhalten.

Die mechanisch verstärkende, regelmäßig periodisch aufgebaute Stützstruktur **03** der verwendeten Leichtbauplatte **02** (vergleiche auch **FIGUR 2****)** weist eine fraktale Gliederung in zumindest zwei fraktalen Ebenen **05, 06** auf. Dabei sind die beiden fraktalen Ebenen **05, 06** auf einer Seite der Leichtbauplatte **02** bündig angeordnet, d.h. sie bilden eine plane Auflagefläche. In der ersten fraktalen Ebene **05** mit einzelnen großen Waben **07** befinden sich innerhalb jeder große Wabe **07** sieben kleinere Waben **08,** die die zweite fraktale Ebene **06** bilden und die eine zusätzliche Versteifung gewährleisten. Weitere Waben innerhalb dieser kleinen Waben **08** würden eine dritte fraktale Ebene bilden. Auch die dritte fraktale Ebene würde dann bündig mit den beiden anderen fraktalen Ebenen **05, 06** abschließen, sodass sie die Auflagefläche der Stützstruktur **03** noch vergrößern würde, ohne das Gewicht bedeutsam zu erhöhen. Die großen und die kleinen Waben **07, 08** bilden Durchbrüche **09.** Die großen Waben **07** der ersten fraktalen Ebene **05** werden von geschlossenen ersten Rippen **10** gebildet, deren Höhe größer ist als die der zweiten Rippen **11** der kleinen Waben **08** in der zweiten fraktalen Ebene **06.** Alle Waben **07, 08** bzw. Rippen **10, 11** berühren die Haut des Unterarms und minimieren dadurch mögliche Irritationsstellen. Der Durchmesser der hautberührenden großen Waben **07** liegt knapp unter 30 mm. Dadurch wird sicher Ödembildung (Gewebeschwellung durch Flüssigkeitseinlagerung) verhindert und trotzdem der größtmögliche Wabendurchmesser mit einer größtmöglichen Materialeinsparung bei gleichzeitig optimaler Stützwirkung genutzt. Mittels der offenen Stützstruktur **03** wird letztendliche eine Leichtbauweise definiert, die eine gleich bleibende bzw. verbesserte Spannungsverteilung und gute Festigkeitseigenschaften, sowie einen höheren Tragekomfort gegenüber bekannten orthopädischen Formstützen aus einfachen ("unechten") Leichtbauplatten ohne hohe Flächenträgheitsmoment erreicht

Zur weiteren Verstärkung kann die fraktale Stützstruktur **03** mehreren fraktalen Ebenen **05, 06** mit einer nicht-fraktalen Struktur, beispielsweise mit einer linearen Rippenstruktur kombiniert werden. Dieser Fall ist aber in der **FIGUR 1** nicht dargestellt. Durch eine Kombination unterschiedlicher Stützstrukturen fraktaler und nicht fraktaler Art kann jede echte Leichtbaukonstruktion ohne zusätzlichen bedeutenden Materialeinsatz optimal ausgesteift und an den jeweiligen Anwendungsfall angepasst werden. Zu weiteren Ausbildungen von fraktalen Stützstrukturen wird auf die deutsche Patentanmeldung DE 10 2007 011 107.1 verwiesen.

Die **FIGUR 2** zeigt die Leichtbauplatte **02** gemäß **FIGUR 1** vor der Umformung mit zwei Detailansichten. Zu erkennen ist die fraktale Stützstruktur **03** mit Durchbrüchen **09,** bestehend aus großen Waben **07** in der ersten fraktalen Ebene **05** mit einem Durchmesser von knapp unter 30 mm und kleinen Waben **08** in der zweiten fraktalen Ebene **06** mit einem entsprechend geringeren Durchmesser. Gut zu erkennen ist in der **FIGUR 2** der bündige Abschluss der beiden fraktalen Ebenen **05, 06** auf einer Seite der Leichtbauplatte **02,** hier gezeigt auf der Rückseite. Diese ist daher die Seite, die bei der späteren Anpassung der Formstütze **01** dem Körper zugewandt ist. Durch die Bündigkeit der beiden fraktalen Ebenen **05, 06** ergibt sich eine gleichmäßige Krafteinleitung über die gesamte Stützstruktur, sodass keine Überbelastungen entstehen können.

Bei der Festlegung dieser fraktalen Stützstruktur **03** wird eine fraktale wabenförmige Lochform (Waben in Waben), nach dem Vorbild der Kieselalge *Isthmia* angewendet. Durch diese Form wird die Möglichkeit geschaffen, einen hohen Prozentanteil der orthopädischen Formstütze **01** völlig offen zu gestalten. Die offene Wabenstruktur bietet zusätzlich - bei ggfs. entsprechend ausgeformtem Polstermaterial - die Gelegenheit, eine vorhandene Wunde zu beobachten, ohne den Verband entfernen zu müssen. Dadurch kann der behandelnde Arzt den Verlauf der Heilung besser verfolgen, ohne zusätzlich Zeit bei der Öffnung und erneuten Anbringung des orthopädischen Formstütze **01** zu verlieren. Neben der hohen Atmungsaktivität der fraktalen Stützstruktur **03** ist ein weiteres vorteilhaftes Kriterium die Materialeinsparung beim verwendeten Werkstoff. Für die orthopädische Formstütze **01** aus PVA für einen Arm ergibt sich ein Gewicht von 62,5g, entsprechend einer etwa 75%igen Gewichtsreduktion gegenüber einem vergleichbaren Stütz- und Verbandssystem.

Durch die wabenförmige fraktale Stützstruktur **03** ergeben sich sehr gute Spannungsverteilungen bei gleichzeitig hoher Steifigkeit der Struktur. Diese Ergebnisse beziehen sich auf die Festigkeit der fraktalen Stützstruktur **03,** die sich aus der Wabenform und der Wandstärke der Rippen **10, 11** ergibt. Durch die zugewiesene fraktale Wabenstruktur bekommt die Leichtbauplatte **02** ein hohes Flächenträgheitsmoment bei geringem Gewicht und zählt damit zu einer echten Leichtbaukonstruktion, vergleichbar mit einem I-Träger.

Die gezeigte fraktale Stützstruktur **03** mit großen und kleinen Waben **07,08** in Kombination mit dem Kunststoff Polyvinylalkohol (PVA unter Zugabe von Formaldehyden) als gewähltem Material stellt eine Ausführungsvariante der Erfindung dar. Es können jedoch auch unterschiedlichste Variationen vorgenommen werden, beispielsweise Variation der Wandstärke, der Tiefe bzw. Höhe der Rippen **10, 11** der Waben **07,08,** der Wabenform oder Integrieren bzw. Ersetzen der Wabenform durch andere Formen, wie beispielsweise Kreise, z:B.: zentrische Formen, wie elliptische Kreise und andere Formen in unterschiedlichen Größen bzw. Durchmessern, eckige Formen (Drei-, Vier-, Rechtecke und mehrfach eckige Formen) sowie Rippen, Schlitze und weitere.

Bei der Verwendung von PVA ergeben sich folgende mit der fraktalen wabenförmigen Stützstruktur **03:** Das Material kann ohne weitere Schutzmaßnahmen mit der Hand aus dem Dampfofen entnommen werden, nachdem es bei ca. 110 °C erwärmt wurde, da es eine nur geringe Wärmekapazität aufweist. Das Anlegen auf die Haut erwies sich nach Aussage eines Probanden als sehr angenehm. Durch die große Oberflächenstruktur des Materials und die Porosität entweicht die Wärme im Material sehr schnell. Nach der Entnahme aus dem Ofen, bleibt dem Personal eine Modellierzeit von weniger als 2 Minuten, die sich durch die atmungsaktive Struktur ergibt.

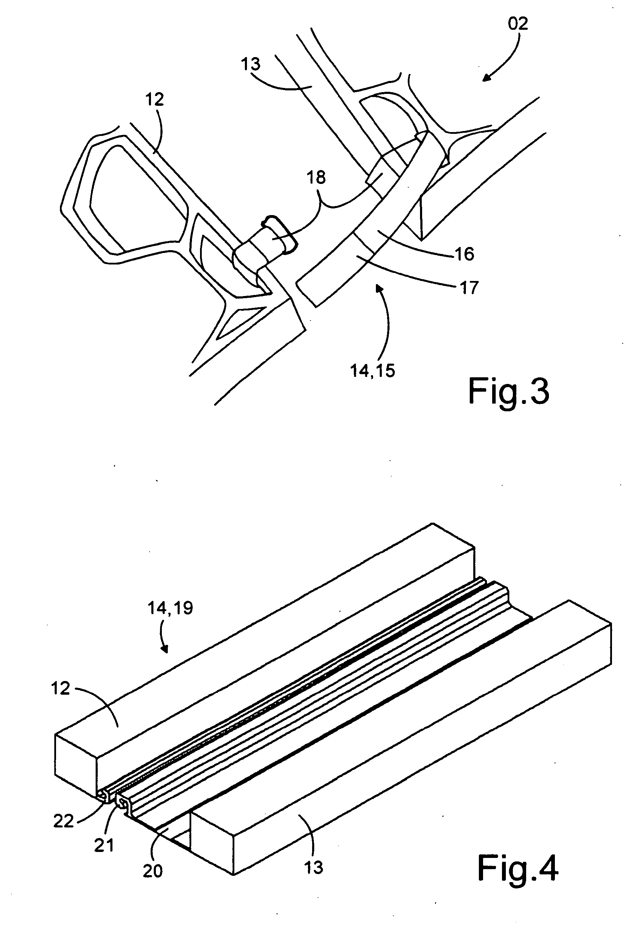

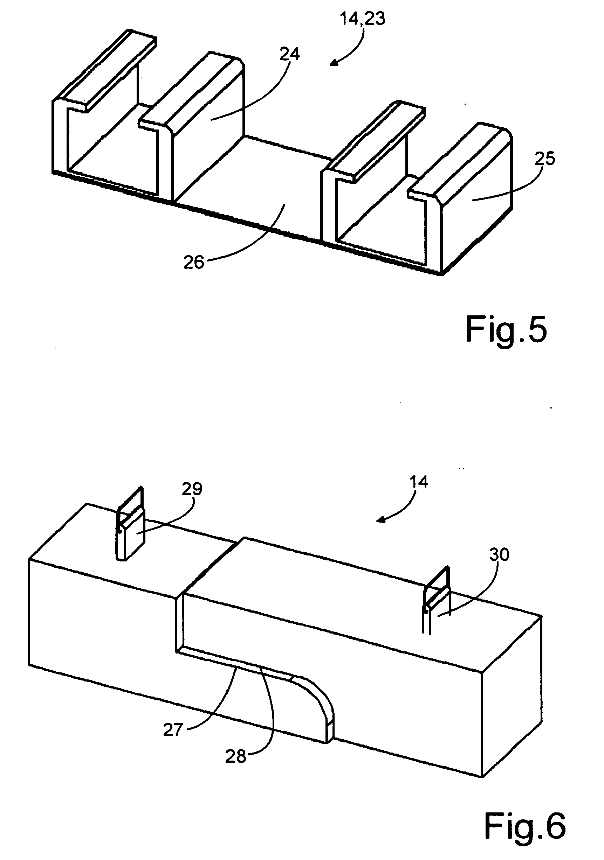

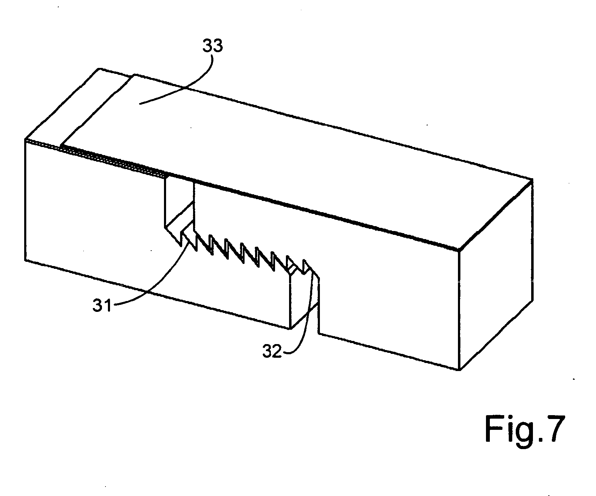

Bei der Umformung werden die beiden Längskanten **12, 13** der Leichtbauplatte **03** einander gegenüberliegend oder überlappend positioniert. Bei der Ruhigstellung z.B. des Armes eines Patienten wird dieser bei der gegenüberliegenden Variante nicht komplett umschlossen, es bleibt ein Längsspalt offen (analog zum Spaltgips). Bei der überlappenden Variante entsteht ebenfalls ein Bewegungsspalt durch eine entsprechende Ausgestaltung der Längskanten **12,13** (vergleiche **FIGUREN 6****,7**). Damit die orthopädische Formstütze **01** Stabilität erlangt und nicht im täglichen Gebrauch ihre Stützwirkung verliert oder sogar vollständig abfällt, ist es erforderlich, die orthopädische Formstütze **01** mittels einer Fixiereinrichtung **14** (vergleiche **FIGUR 3****)** zu fixieren. Dabei kann die Fixiereinrichtung **14** unterschiedlich ausgeführt sein.

Die **FIGUR 3** zeigt perspektivisch ein Detail eines Klettverschlusses **15** als Fixiereinrichtung **14.** Bei dieser Variante wird die Leichtbauplatte **02** mit mehreren Klettverschlüssen **15** mit einem feinen Klettband **16** und einem rauen Klettband **17** über zwei C-förmige Haken **18** fixiert. Mehrere C-förmige Haken **16** sind an den beiden Längskanten **12, 13** der umgeformten Leichtbauplatte **02** befestigt. Diese Verschlussart eignet sich gut für eine normale Leichtbauplatte **02,** die bei leichten Frakturen oder Prellungen für eine Ruhigstellung eingesetzt wird.

Die **FIGUR 4** zeigt perspektivisch ein Detail eines Hakenverschlusses **19** mit einem elastischen Bindeneinsatz **20** als Fixiereinrichtung **14.** Diese kann in kurze Abschnitte geteilt sein, sodass mehrere Hakenverschlüsse **19** entlang der Längskanten **12, 13** vorgesehen sein müssen. Der Bindeneinsatz **20** kann aber auch über die gesamte Erstreckung der Längskanten **12, 13** vorgesehen sein (wie in **FIGUR 4** gezeigt) und somit den Längsspalt vollständig überbrücken. Der Bindeneinsatz **20** ist an der einen Längskante **13** angebracht und weist an seinem anderen Ende einen ersten Haken **21** auf, der zum Eingriff mit einem zweiten Haken **22,** der an der anderen Längskante **12** angeordnet ist, gelangt.

Die **FIGUR 5** zeigt perspektivisch einen Clipverschluss **23.** Hierbei handelt es sich um eine Fixiereinrichtung **14,** die separat an der orthopädischen Formstütze **01** angebracht wird. Zwei Clipse **24, 25** werden über einen Bindeneinsatzabschnitt **26** zusammen gehalten. Nach dem Anlegen der vorgeformten orthopädischen Formstütze **01** an den Arm werden die Seiten des Clipse **24, 25** mit der offen Seite nach oben schauend an die offenen Strukturen der Leichtbauplatte **02** an den beiden Längskanten **12, 13** einfach eingeclipst. Diese Fixiereinrichtung **14** arbeitet ähnlich wie die Krallenverschlüsse bei elastischen Binden. Der Vorteil des Clipverschlusses **23** ist darin zu sehen, dass er nicht bei der Fertigung der orthopädischen Formstütze **01** integriert werden muss, wodurch die Produktionskosten gesenkt werden können.

Die **FIGUREN 6** und **7** zeigen perspektivisch zwei verschiedene Fixiereinrichtungen **14** für den Fall, dass die Längskanten **12, 13** nach der Umformung der Leichtbauplatte **02** mit Passform übereinander liegen. Die **FIGUR 6** zeigt eine Passform mit glatten Längskanten **27, 28**. Die Fixiereinrichtung **14** kann dann beispielsweise von einem Klettverschluss gebildet werden, der in entsprechende C-förmige Haken **29, 30** eingreift. Bei einer Ausbildung der Passform mit gezahnten Längskanten **31, 32** gemäß **FIGUR 7** erfolgt eine Fixierung bereits über das Ineinandergreifen der Verzahnung. Ein Auseinanderrutschen der Verzahnung kann wiederum durch einen Klettverschluss **33,** der beispielsweise aufgeklebt oder aufgetackert ist, verhindert werden.

Bei Gips- und Castverbänden kommt es nach längerer Tragezeit zu einer Abschwellung der Verletzung, die zu einer Lockerung des Verbandes führt. Unter Umständen muss dann der Verband erneuert werden. Mit dem Klettverschluss **33** bei überlappenden Längskanten **27, 28, 31, 32** ist eine Anpassung des orthopädischen Stützform **01** an den abschwellenden Arm ohne weiteres möglich. Es ergeben sich deutliche Vorteile für Patient und Arzt durch eine angenehmere Behandlung und durch eine Kostenreduzierung.

### Bezugszeichenliste

- **01**: orthopädische Formstütze
- **02**: Leichtbauplatte
- **03**: fraktale Stützstruktur
- **04**: Durchbruch
- **05**: fraktale Ebene (Hauptebene)
- **06**: fraktale Ebene (erste Unterebene)
- **07**: großen Wabe (in 05)
- **08**: kleine Wabe (in 06)
- **09**: Durchbruch
- **10**: Rippe (in 05)
- **11**: Rippe (in 06)
- **12**: erste Längskante
- **13**: zweite Längskante
- **14**: Fixierungssystem
- **15**: Klettverschluss
- **16**: feines Klettband
- **17**: raues Klettband
- **18**: C-förmiger Haken
- **19**: Hakenverschluss
- **20**: Bindeneinsatz
- **21**: erster Haken
- **22**: zweiter Haken
- **23**: Clipverschluss
- **24**: Clip
- **25**: Clip
- **26**: Bindeneinsatzabschnitt
- **27**: glatte Längskante (Passform)
- **28**: glatte Längskante (Passform)
- **29**: C-förmiger Haken
- **30**: C-förmiger Haken
- **31**: gezahnte Längskante (Passform)
- **32**: gezahnte Längskante (Passform)
- **33**: Klettverschluss

## Patentansprüche

1. Orthopädische Formstütze zur nicht-chirurgischen Behandlung von Knochen, Gelenken, Sehnen oder Bändern, hergestellt aus einer aus einem thermisch umformbaren Material bestehenden, Durchbrüche aufweisenden Leichtbauplatte mit einer mechanisch verstärkenden, regelmäßig periodisch aufgebauten Stützstruktur unter Nutzung einer mechanisch verstärkenden, regelmäßig periodisch aufgebauten Stützstruktur nach biologischem Vorbild, die an das zu behandelnde Körperteil angepasst und mittels einer Fixiereinrichtung fixiert wird,
**gekennzeichet durch**
eine fraktale Gliederung der Stützstruktur (03) in zumindest zwei ineinander liegenden fraktalen Ebenen (05, 06) mit einer selbstähnlichen Wiederholung des Verlaufs der Stützkontur der Stützstruktur (03) in sich selbst zumindest noch ein weiteres Mal, wobei jede fraktale Ebene (05, 06) durch eine Verkleinerung der Stützstruktur (03) definiert ist, fließende Übergänge zwischen den fraktalen Ebenen vorliegen (5/5) und die fraktalen Ebenen (05, 06) auf der der zu behandelnden Körperstelle zugewandten Seite der Leichtbauplatte (02) bündig abschließen.

2. Orthopädische Formstütze nach Anspruch 1,
**gekennzeichnet durch**
eine Größe aller Durchbrüche (04, 09) unterhalb von 30 mm.

3. Orthopädische Formstütze nach Anspruch 1 oder 2,
**gekennzeichnet durch**
eine fraktale Gliederung der Stützstruktur (03) unter Nutzung der skalierten natürlichen Stützstrukturen der biomineralisierenden Exoskelette mariner Mikroalgen oder Ammoniten.

4. Orthopädische Formstütze nach einem der Ansprüche 1 bis 3,
**gekennzeichnet durch**
eine fraktale Gliederung der Stützstruktur (03) aus Stützrippen mit geradem oder gewelltem verlauf.

5. Orthopädische Formstütze nach einem der Ansprüche 1 bis 4,
**gekennzeichnet durch**
**durch** eine Kombination der fraktal gegliederten Stützstruktur (03) mit einer nicht fraktal gegliederten Stützstruktur.

6. Orthopädische Formstütze nach einem der Ansprüche 1 bis 5,
**gekennzeichnet durch**
eine Leichtbauplatte (02) aus einem Kunststoff, der thermoplastische und duroplastische Eigenschaften aufweist.

7. Orthopädische Formstütze nach Anspruch 6,
**gekennzeichnet durch**
eine Faserverstärkung des Kunststoffes.

8. Orthopädische Formstütze nach einem der Ansprüche 1 bis 7,
**gekennzeichnet durch**
eine mittels Hohlkugeln versteifte Leichtbauplatte (02)..

9. Orthopädische Formstütze nach einem der Ansprüche 1 bis 8,
**gekennzeichnet durch**
ein hautverträgliches Polstersystem auf der Unterseite der Stützstruktur (03).

10. Orthopädische Formstütze nach Anspruch 11,
**gekennzeichnet durch**
eine Ausbildung des Polstersystems als textiles oder aufblasbares System.

11. Orthopädische Formstütze nach Anspruch 7 oder 8,
**gekennzeichnet durch**
eine Anpassung des Polstersystems an die durchbrochene Leichtbauplatte (02) in der Form, dass die Durchbrüche (04, 09) offen bleiben.

12. Orthopädische Formstütze nach einem der Ansprüche 1 bis 11,
**gekennzeichnet durch**
eine Kombination mit Kompressionsmaterial auf der Oberseite der Stützstruktur (03).

13. Orthopädische Formstütze nach einem der Ansprüche 1 bis 12,
**gekennzeichnet durch**
einen Längsspalt, der **durch** die beiden Längskanten (12, 13) der Leichtbauplatte (02) nach der Anpassung gebildet ist.

14. Orthopädische Formstütze nach Anspruch 13,
**gekennzeichnet durch**
gegenüberliegende Längskanten (12, 13), die **durch** die Fixiereinrichtung (14) zusammengehalten werden.

15. Orthopädische Formstütze nach Anspruch 14,
**gekennzeichnet durch**
eine Ausbildung der Fixiereinrichtung (14) als den Längsspalt überbrückender Klettverschluss (15) oder als Haken- oder Clipverschluss (19, 23) mit einem elastischen Bindeneinsatz (20).

16. Orthopädische Formstütze nach einem der Ansprüche 1 bis 12,
**gekennzeichnet durch**
mit Passform übereinander liegende Längskanten (27, 28, 31, 32), die **durch** die Fixiereinrichtung (14) zusammengehalten werden.

17. Orthopädische Formstütze nach Anspruch 16,
**gekennzeichnet durch**
eine Ausbildung der Passform mit glatten (27, 28) oder gezahnten (31, 32) Längskanten, wobei die Fixiereinrichtung (14) als Klettverschluss (15) entlang des Längsspaltes ausgebildet ist.

18. Orthopädische Formstütze nach einem der Ansprüche 1 bis 17,
**gekennzeichnet durch**
beliebige Farbgebung für die Leichtbauplatte (02) und/oder das Polstersystem.

## Claims

1. Orthopaedic shaped support for the non-surgical treatment of bones, joints, tendons or ligaments, comprising a lightweight structural plate made of a thermally shapeable material with apertures and with a mechanically strengthening, regularly periodically built-up support structure using a mechanically strengthening, regularly periodically built-up support structure based on a biological model, which is fitted to the body part to be treated and attached by means of a fixing device,
**characterised by**
a fractal separation of the support structure (03) into at least two fractal planes (05, 06) located within each other, with a self-similar repetition of the course of the support contour of the support structure (03) in itself at least one more time, whereby each fractal plane (05, 06) is defined by a reduction of the support structure (03), fluid transitions are present between the fractal planes (5/5) and the fractal planes (05, 06) provide a flush finish on the side of the lightweight structural plate (02) facing the part of the body to be treated.

2. Orthopaedic shaped support in accordance with claim 1,
**characterised by**
a size of all apertures (04, 09) of under 30 mm.

3. Orthopaedic shaped support in accordance with claim 1 or 2,
**characterised by**
a fractal separation of the support structure (03) using the scaled natural support structures of the biomineralising exoskeleton of marine micro-algae or ammonites.

4. Orthopaedic shaped support in accordance with any one of claims 1 to 3,
**characterised by**
a fractal separation of the support structure (03) of straight or undulating support ribs.

5. Orthopaedic shaped structure in accordance with any one of claims 1 to 4,
**characterised by**
a combination of the fractally separated support structure (03) with a non-fractally separated support structure.

6. Orthopaedic shaped support in accordance with any one of claims 1 to 5,
**characterised by**
a lightweight structural plate (02) made of a plastic exhibiting thermoplastic and duroplastic properties.

7. Orthopaedic shaped support in accordance with claim 6,
**characterised by**
fibre-reinforcement of the plastic.

8. Orthopaedic shaped support in accordance with any one of claims 1 to 7,
**characterised by**
a lightweight structural plate (02) stiffened by means of hollow spheres.

9. Orthopaedic shaped support in accordance with any one of claims 1 to 8,
**characterised by**
a skin-compatible cushioning system on the underside of the support structure (03).

10. Orthopaedic shaped support in accordance with claim 11,
**characterised by**
the cushioning system being designed as a textile or inflatable system.

11. Orthopaedic shaped support in accordance with claim 7 or 8,
**characterised by**
an adaptation of the cushioning system to the perforated lightweight structural plate (02) so that apertures (04, 09) remain open.

12. Orthopaedic shaped support in accordance with any one of claims 1 to 11,
**characterised by**
a combination with compression material on the upper side of the support structure (03).

13. Orthopaedic shaped support in accordance with any one of claims 1 to 12,
**characterised by**
a longitudinal gap formed by the longitudinal edges (12, 13) of the lightweight structural plate (02) after adaptation.

14. Orthopaedic shaped support in accordance with claim 13,
**characterised by**
longitudinal edges (12, 13) facing each other that are held together by fixing means (14).

15. Orthopaedic shaped support in accordance with claim 14,
**characterised by**
the fixing device (14) being designed as a Velcro closure (15) bridging the longitudinal gap, or as a hook or clip closure (19, 23) with an elastic binding insert (20).

16. Orthopaedic shaped support in accordance with an one of claims 1 to 12,
**characterised by**
perfect fitting longitudinal edges (27, 28, 31, 32), lying on top of each other that are held together by the fixing device (14).

17. Orthopaedic shaped support in accordance with claim 16,
**characterised by**
perfect fitting with smooth (27, 28) or indented (31, 32) longitudinal edges, whereby the fixing device (14) is in the form of a Velcro closure (15) along the longitudinal gap.

18. Orthopaedic shaped support in accordance with any one of claims 1 to 17,
**characterised by**
the lightweight structural plate (02) and/or cushioning system being of any colour.

## Revendications

1. Support moulé orthopédique pour le traitement non chirurgical d'os, d'articulations, de tendons ou de ligaments, fabriqué à partir d'une plaque à structure légère composée d'un matériau déformable thermiquement et présentant des percées, dotée d'une structure de support assurant un renfort mécanique et réalisée régulièrement en périodes en utilisant une structure de support assurant un renfort mécanique et réalisée régulièrement en périodes suivant un modèle biologique, qui est adapté à la partie du corps à traiter et est fixée au moyen d'un dispositif de fixation,
**caractérisé par**
une configuration fractale de la structure de support (03) dans au moins deux plans fractals imbriqués (05, 06) avec une répétition autosimilaire de l'évolution du contour de support de la structure de support (03) en soi au moins encore une autre fois, chaque plan fractal (05, 06) étant défini par une réduction de la structure de support (03), sachant qu'il y a des transitions fluides entre les plans fractals (5/5) et que les plans fractals (05, 06) se terminent à fleur sur le côté tourné vers le point du corps à traiter de la plaque à structure légère (02).

2. Support moulé orthopédique selon la revendication 1,
**caractérisé par**
une dimension de toutes les percées (04, 09) inférieure à 30 mm.

3. Support moulé orthopédique selon la revendication 1 ou 2,
**caractérisé par**
une configuration fractale de la structure de support (03) en utilisant les structures de support naturelles échelonnées de l'exosquelette biominéralisant d'algues marines ou d'ammonites.

4. Support moulé orthopédique selon une des revendications 1 à 3,
**caractérisé par**
une configuration fractale de la structure de support (03) composée de nervures de support à extension rectiligne ou ondulée.

5. Support moulé orthopédique selon une des revendications 1 à 4,
**caractérisé par**
une combinaison de la structure de support à configuration fractale (03) avec une structure de support dépourvue de configuration fractale.

6. Support moulé orthopédique selon une des revendications 1 à 5,
**caractérisé par**
une plaque à structure légère (02) faite d'une matière plastique qui présente des propriétés thermoplastiques et duroplastiques.

7. Support moulé orthopédique selon la revendication 6,
**caractérisé par**
un renforcement par des fibres de la matière plastique.

8. Support moulé orthopédique selon une des revendications 1 à 7,
**caractérisé par**
une plaque à structure légère (02) renforcée par des billes creuses.

9. Support moulé orthopédique selon une des revendications 1 à 8,
**caractérisé par**
un système de rembourrage toléré par la peau sur la face inférieure de la structure de support (03).

10. Support moulé orthopédique selon la revendication 11,
**caractérisé par**
une réalisation du système de rembourrage sous forme de système textile ou gonflable.

11. Support moulé orthopédique selon la revendication 7 ou 8,
**caractérisé par**
une adaptation du système de rembourrage à la plaque à structure légère percée (02) d'une forme telle que les percées (04, 09) restent ouvertes.

12. Support moulé orthopédique selon une des revendications 1 à 11,
**caractérisé par**
une combinaison avec du matériau de compression sur la face supérieure de la structure de support (03).

13. Support moulé orthopédique selon une des revendications 1 à 12,
**caractérisé par**
une fente longitudinale qui est formée par les deux bords longitudinaux (12, 13) de la la plaque à structure légère (02) après l'adaptation.

14. Support moulé orthopédique selon la revendication 13,
**caractérisé par**
des bords longitudinaux opposés (12, 13) qui sont maintenus ensemble par le dispositif de fixation (14).

15. Support moulé orthopédique selon la revendication 14,
**caractérisé par**
une réalisation du dispositif de fixation (14) sous forme de fermeture velcro recouvrant la fente longitudinale (15) ou sous forme de fermeture à crochet ou à clip (19, 23) avec une garniture de bandage élastique (20).

16. Support moulé orthopédique selon une des revendications 1 à 12,
**caractérisé par**
des bords longitudinaux superposés par une forme d'ajustement (27, 28, 31, 32) et qui sont maintenus ensemble par le dispositif de fixation (14).

17. Support moulé orthopédique selon la revendication 16,
**caractérisé par**
une conformation de la forme d'ajustement avec des bords longitudinaux lisses (27, 28) ou dentés (31, 32), le dispositif de fixation (14) étant réalisé sous forme de fermeture velcro (15) le long de la fente longitudinale.

18. Support moulé orthopédique selon une des revendications 1 à 17,
**caractérisé par**
une coloration quelconque pour la plaque à structure légère (02) et/ou le système de rembourrage.
